# EUROPEAN PATENT APPLICATION

(11) **EP 4 302 800 A1**
(43) Date of publication of application: **10.01.2024**
(21) Application number: 22775663.2
(22) Date of filing: 23.03.2022
(51) Int. Cl.: A61M 5/142, A61M 5/315

(54) **CONTINUOUS ADMINISTRATION DEVICE**

(30) Priority: 25.03.2021 JP 2021051366
(71) Applicant: TERUMO Kabushiki Kaisha, Tokyo 151-0072 (JP)
(72) Inventor: MITSUOKA, Takumi, Ashigarakami-gun, Kanagawa 259-0151 (JP); YOSHIKAWA, Hiroki, Ashigarakami-gun, Kanagawa 259-0151 (JP); ITO, Hiroshi, Showa-cho, Yamanashi 409-3853 (JP); HYAKKAN, Yusuke, Ashigarakami-gun, Kanagawa 259-0151 (JP); ARINOBE, Manabu, Ashigarakami-gun, Kanagawa 259-0151 (JP); YAMAMOTO, Satoshi, Ashigarakami-gun, Kanagawa 259-0151 (JP)
(74) Representative: Casalonga
(86) International application number: PCT/JP2022/013404
(87) International publication number: WO 2022/202881

(57) **Abstract**

A continuous administration device includes a cylindrical main body portion having an inner cavity, a plug slidable in the inner cavity in a liquid-tight manner, a pressing portion disposed on an upstream side with respect to the plug in the inner cavity and pressing the plug to a downstream side, and a substance to be administered stored on the downstream side with respect to the plug in the inner cavity and released into a living body by the plug sliding to the downstream side, and is indwelled in the living body for sustained release of the substance to be administered. The continuous administration device includes a sliding solid coating layer formed with a silicone-based resin composition or a fluorine-based resin film for reducing initial and normal sliding resistance of the plug, on at least one of an outer surface of the plug or a region where the plug slides in the inner cavity.

## Description

### Technical Field

The present invention relates to a continuous administration device that continuously administers a substance to be administered such as a drug solution to an administration target in a state where the device is indwelled in a living body.

### Background Art

JP 2020-23562 A discloses an osmotic delivery device that is a continuous administration device that continuously administers (sustained release for a long period of time) a drug that is a substance to be administered in a state where the device is embedded in a living body for a long period of time (at least about three weeks to several months).

The device of JP 2020-23562 A is roughly constituted with a hollow container (main body portion), a piston (plug) slidably held in the container, an osmotic engine (pressing portion) serving as a drive source for pressing the piston, a semipermeable membrane (liquid permeation portion) that is disposed on a proximal end side of the container and allows a liquid component in a body fluid (extracellular fluid) to permeate the osmotic engine, and a delivery orifice (release portion) that is disposed on a distal end side of the container and releases a drug pressed by the piston. The container is partitioned into two spaces by the piston, and includes a drug containing space (second space) positioned on the distal end side from the piston, of the container, and an osmotic preparation containing space (first space) positioned on a proximal end side from the piston, of the container, which is an opposite side of the second space.

The device of JP 2020-23562 A is constituted to be able to expand an osmotic preparation by the liquid component in the body fluid that has permeated the semipermeable membrane, slide the piston toward the distal end side as an internal pressure of the first space increases due to expansion of the osmotic preparation, and release the drug contained in the second space into the living body, using the principle of osmotic pressure.

### Citation List

### Patent Literature

Patent Literature 1: JP 2020-23562

### Summary of invention

### Technical Problem

The device of JP 2020-23562 A is intended to be indwelled in a living body and continuously administer a drug stored in a limited space over a long period of time. Thus, in the device of JP 2020-23562 A, it is necessary to slide the piston at an extremely low speed (as an example, 0.01 mm/min or less) as compared with an administration rate (as an example, 200 mm/min) at the time of general bolus administration. Thus, in the continuous administration device, sliding resistance of the piston is an important factor in controlling a drug administration period.

By the way, in the device of JP 2020-23562 A, the piston is slid by the internal pressure increased by expansion of the osmotic engine, and thus, discharge start time of the drug by the piston depends on sliding resistance of the piston. Further, in the device of JP 2020-23562 A, it takes about 24 to 48 hours to start administration (release) of the substance to be administered. Thus, the device of JP 2020-23562 A has a problem that it takes time to express an effect by the drug.

As a countermeasure against such a problem, it is conceivable to apply silicone oil as a lubricant to an outer peripheral surface of the piston in order to reduce sliding resistance of the piston. However, as apparent in Comparative Example 1 of the present invention described later, it still takes time to start discharging the silicone oil. This is because a time lag occurs from when force for moving starts to be applied to the plug until when the plug actually starts moving. This time lag occurs because the silicone oil is liquid, and thus, the silicone oil applied to an inner wall surface on which the plug or the plug of the cylindrical main body portion slides is pushed away by the plug, and
the plug directly adheres to the inner wall surface, which may cause extremely large sliding resistance until sliding is started. In addition, interaction with a lubricant such as silicone oil may occur depending on a drug solution to be administered, and if the drug solution is stored for a long period of time after being filled, the drug solution may be altered by the interaction. It is therefore difficult to say that the silicone oil is an appropriate material as a lubricant for a piston of a continuous administration device.

At least one embodiment of the present invention has been made in view of the above circumstances, and specifically, it is an object of the present invention to provide a continuous administration device capable of reducing initial sliding resistance and normal sliding resistance of a plug and accelerating expression of an effect by an administered substance.

### Solution to Problem

A continuous administration device according to the present embodiment includes: a cylindrical main body portion having an inner cavity; a plug slidably disposed on a plug that is slidable in the inner cavity in a liquid-tight manner; a pressing portion disposed on an upstream side with respect to the plug in the inner cavity and pressing the plug toward a downstream side; and a substance to be administered stored on the downstream side with respect to the plug in the inner cavity and released into a living body by the plug sliding toward the downstream side, and is indwelled in the living body for sustained release of the substance to be administered. The continuous administration device includes a sliding solid coating layer for reducing initial sliding resistance and normal sliding resistance of the plug, on at least one of an outer surface of the plug or a region where the plug slides in the inner cavity.

### Advantageous Effects of Invention

According to at least one embodiment of the present invention, it is possible to reduce initial sliding resistance and normal sliding resistance of a plug and to accelerate expression of an effect by an administered substance.

### Brief Description of Drawings

FIG. 1 is a schematic perspective view of a continuous administration device according to the present embodiment;
FIG. 2 is a partial cross-sectional view of the continuous administration device according to the present embodiment;
FIG. 3A is a partial perspective view illustrating an operation example of the continuous administration device according to the present embodiment;
FIG. 3B is a partial perspective view illustrating an operation example of the continuous administration device according to the present embodiment;
FIG. 3C is a partial perspective view illustrating an operation example of the continuous administration device according to the present embodiment;
FIG. 3D is a partial perspective view illustrating an operation example of the continuous administration device according to the present embodiment; and
FIG. 4 is a view illustrating a modified example of a release portion of the continuous administration device according to the present embodiment.

### Description of Embodiments

Hereinafter, a mode for carrying out the present invention will be described in detail with reference to the drawings. An embodiment herein is illustrated to embody technical idea of the present invention and does not limit the present invention. Furthermore, other embodiments, examples, operation techniques, and the like, that can be implemented by those skilled in the art without departing from the gist of the present invention are all included in the scope and gist of the present invention and included in the invention described in the claims and the scope of equivalents thereof.

Moreover, for convenience of illustration and ease of understanding, the drawings attached to the present specification may schematically illustrate the present invention by changing a scale, an aspect ratio, a shape, and the like, from actual ones as appropriate, but are merely examples, and do not limit interpretation of the present invention.

Note that, in the following description, ordinal numerals such as "first" and "second" will be given, but are used for convenience and do not define any order unless otherwise specified.

A continuous administration device 100 according to the present embodiment is a device for continuously administering (sustained release) a substance to be administered X such as a drug to an administration target such as a living body for a long period of time (at least three weeks to several months and even several years). The substance to be administered X from the continuous administration device 100 is a fluid composition that can express a predetermined effect by long-term sustained release to the living body that is the administration target and can be continuously released from the device. The substance to be administered X is, for example, a drug (liquid preparation) intended for treatment of a predetermined disease. The drug includes a drug. The drug can be any physiologically or pharmacologically active substance, in particular one known to be delivered to a human or animal body. The drug includes, but is not limited to, a drug acting on a peripheral nerve, an adrenergic receptor, a cholinergic receptor, a skeletal muscle, a cardiovascular system, a smooth muscle, a vascular system, a synoptic site, a nerve exchanger junction site, an endocrine and hormonal system, an immune system, a reproductive system, a skeletal system, a local hormonal system, a digestive and excretory system, a histamine system, or a central nervous system. In addition, the drug includes, but is not limited to, a drug to be used for treatment of infectious diseases, chronic pain, diabetes, autoimmune diseases, endocrine diseases, metabolic abnormalities, and rheumatic diseases. In addition, the drug includes, but is not limited to, peptide, protein, polypeptide (for example, enzyme, hormone, cytokine), nucleic acid, nucleoprotein, polysaccharide, glycoprotein, lipoprotein, cell, steroid, analgesic, local anesthetic, antibiotic formulation, antiinflammatory corticosteroid, eye drops, other small molecules for pharmaceutical use, or synthetic analogs of these species, and mixtures thereof, and the like. As an example, the continuous administration device 100 is indwelled in a subcutaneous tissue of a patient and used.

The continuous administration device in related art as disclosed in JP 2020-23562 A has a problem that it takes time until the substance to be administered X is released after the device is indwelled in the living body, and thus, it takes time to express an effect by the administered substance X. The present inventors considered application of silicone oil used as a lubricant for the purpose of improving slidability between an inner surface of a main body portion 10 and a plug (piston) 40 in order to reduce initial sliding resistance and normal sliding resistance of the continuous administration device 100 and shorten a period until drug discharge start time (effect expression time), but the initial sliding resistance was large and shortening of the period until the drug discharge start time was insufficient. As a result of further studies, the present inventors have succeeded in sufficiently reducing initial sliding resistance and normal sliding resistance, particularly the initial sliding resistance, by adopting a configuration in which a sliding solid coating layer 60 is interposed between the inner surface of the main body portion 10 and the plug (piston) 40.

### <Configuration>

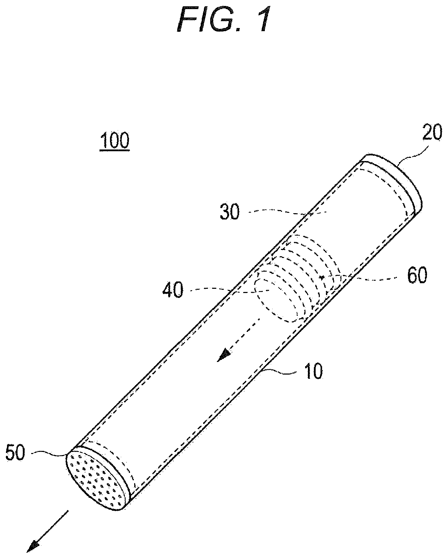

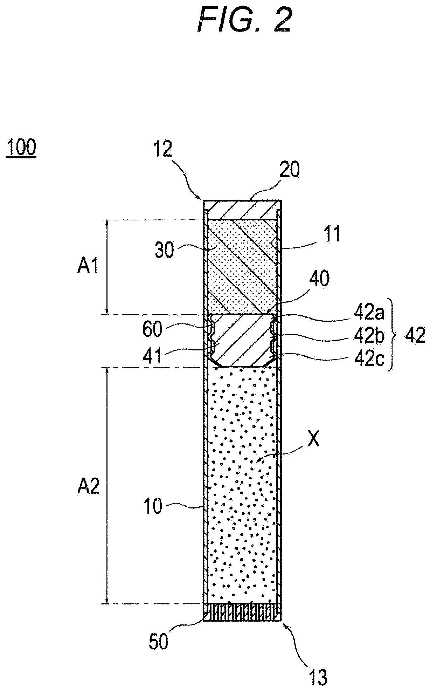

As illustrated in FIG. 1 or FIG. 2, the continuous administration device 100 generally includes a main body portion 10, a liquid permeation portion 20, a pressing portion 30, a plug 40, and a release portion 50. In addition, the continuous administration device 100 includes a sliding solid coating layer 60 for reducing initial sliding resistance and normal sliding resistance of the plug 40 between an inner peripheral surface of an inner cavity 11 of the main body portion 10 and an outer peripheral surface of the plug 40.

The main body portion 10 is constituted with a hollow cylindrical member constituting a housing of the continuous administration device 100. The main body portion 10 has a proximal portion 12 located on an upstream side (left side in FIG. 1) into which a liquid component in a body fluid (extracellular fluid) flows and a distal portion 13 located on a downstream side (right side in FIG. 1) from which the substance to be administered X is released to the living body. The liquid permeation portion 20, the pressing portion 30, the plug 40, and the release portion 50 are arranged in order from the proximal portion 12 toward the distal portion 13 of the main body portion 10.

The main body portion 10 has a first space A1 and a second space A2 partitioned by the plug 40. The first space A1 is partitioned by the liquid permeation portion 20 and the plug 40, and is a space in which the pressing portion 30 is stored. The second space A2 is a space partitioned by the plug 40 and the release portion 50 and in which the substance to be administered X is stored. At least part of the second space A2 constitutes a region where the plug 40 slides.

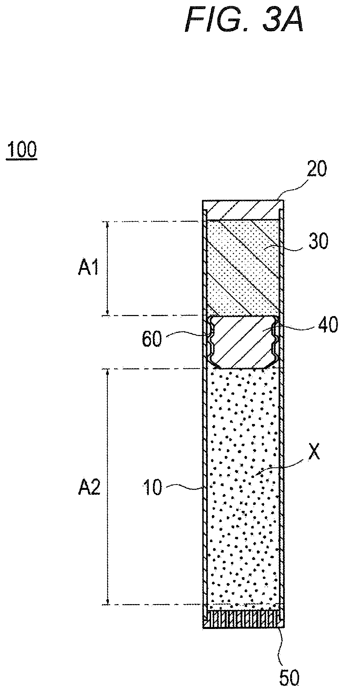

Spaces of the first space A1 and the second space A2 are enlarged or reduced as the plug 40 slides before and after the continuous administration device 100 is driven (see FIGS. 3A to 3D). In other words, in the first space A1, if the continuous administration device 100 starts to be driven, the plug 40 slides toward the downstream side by the pressing portion 30, and an interval gradually increases. Further, in the second space A2, if the continuous administration device 100 starts to be driven, the plug 40 slides toward the downstream side by the pressing portion 30, and the interval gradually narrows.

As a constituent material of the main body portion 10, a resin material (acrylonitrile polymers, halogenated polymers, polyimide, polysulfone, polycarbonate, polyethylene, polypropylene, polyvinyl chloride-acrylic acid copolymer, polycarbonate-acrylonitrile-butadienestyrene, polystyrene, and the like) or a metal material (stainless steel, titanium, nickel, aluminum, vanadium, platinum, tantalum, gold, and alloys thereof, and gold plated alloy iron, platinum plated alloy iron, cobalt chromium alloy, titanium nitride-coated stainless steel, and the like) which is non-invasive or minimally invasive to a living body and known in the medical field can be applied.

The liquid permeation portion 20 is disposed on the proximal end side of the main body portion 10, isolates the living body from inside of the continuous administration device 100, and permeates only a liquid component contained in a body fluid in the living body. The liquid permeation portion 20 is constituted with a member having a solid-liquid separation function capable of permeating only a liquid component in a body fluid. As an example, a semipermeable membrane including a plasticized cellulose-based material, reinforced polymethyl methacrylates (PMMA) such as hydroxyl ethyl methacrylate (HEMA), and elastomer materials such as polyurethanes and polyamides, polyether-polyamide copolymers, and thermoplastic copolyesters can be applied as the liquid permeation portion 20.

The pressing portion 30 is located on the downstream side with respect to the liquid permeation portion 20 in the main body portion 10 and presses the plug 40 toward the downstream side. In the present embodiment, the pressing portion 30 is an osmotic engine that expands by being permeated by the liquid component that has permeated the liquid permeation portion 20 using the principle of osmotic pressure. The pressing portion 30 is gradually expanded by the liquid component that has permeated the liquid permeation portion 20 and slides the plug 40 toward the downstream side by pressing action caused by increase in an internal pressure in the first space A1 due to the expansion.

A constituent material, a size, and the like, of the pressing portion 30 can be appropriately determined so as to achieve a pressing speed (that is, an expansion speed of the pressing portion 30) of the plug 40 according to use conditions (a sustained release period, a release amount per unit time, and the like, of the substance to be administered X). The continuous administration device 100 can sustainably release the drug for a long period of time over several years depending on the composition of the osmotic engine constituting the pressing portion 30, a thickness of the semipermeable membrane constituting the liquid permeation portion 20, and the like.

The plug 40 includes a columnar base portion 41 and an annular protruding portion 42 protruding from a radially outer periphery of the base portion 41. The plug 40 is pressed by the pressing portion 30 and moves to the downstream side to push out the substance to be administered X stored in the second space A2 toward the release portion 50. The plug 40 is located on the downstream side with respect to the pressing portion 30 in the main body portion 10, has an outer peripheral surface in liquid-tight contact with the inner peripheral surface of the inner cavity 11 of the main body portion 10 and is slidably disposed in the inner cavity 11.

In the present embodiment, the protruding portion 42 includes a first protruding portion 42a provided on an outer surface on the proximal end side of the base portion 41, a second protruding portion 42b provided substantially in the middle of the base portion 41, and a third protruding portion 42c provided on the outer surface on the distal end side of the base portion 41. An apex portion protruding outermost of the protruding portion 42 abuts on the inner peripheral surface of the inner cavity 11 of the main body portion 10. The first protruding portion 42a, the second protruding portion 42b, and the third protruding portion 42c seal between the inner peripheral surface of the inner cavity 11 and the first protruding portion 42a, the second protruding portion 42b, and the third protruding portion 42c so that the substance to be administered X does not leak to the proximal end side with respect to the plug 40.

Although the protruding portion 42 includes the first protruding portion 42a, the second protruding portion 42b, and the third protruding portion 42c, the number and arrangement positions thereof are not particularly limited. In addition, it is also possible to employ a configuration where the plug 40 does not include the protruding portion 42.

As a constituent material of the plug 40, the plug 40 is constituted with a flexible material that has adhesion (liquid tightness) to the inner peripheral surface of the inner cavity 11 of the main body portion 10.

The flexible material is preferably an elastic material, and examples of the elastic material include various rubber materials (in particular, those subjected to vulcanization treatment) such as natural rubber, isoprene rubber, butyl rubber, chloroprene rubber, nitrile-butadiene rubber, styrene-butadiene rubber, and silicone rubber, styrene-based elastomers, hydrogenated styrene-based elastomers, and styrene-based elastomers mixed with polyolefins such as polyethylene, polypropylene, polybutene, and α-olefin copolymers, oils such as liquid paraffin and process oil, and powder inorganic substances such as talc, cast, and mica. Furthermore, a polyvinyl chloride-based elastomer, an olefin-based elastomer, a polyester-based elastomer, a polyamide-based elastomer, a polyurethane-based elastomer, or a mixture thereof can be used as the constituent material. As the constituent material, in particular, a diene-based rubber, a styrene-based elastomer, or the like can be applied from the viewpoint of having elastic characteristics and enabling γ-ray sterilization, electron beam sterilization, and highpressure steam sterilization. The plug 40 only requires to be slidable in the inner cavity 11 in a liquid-tight manner. Thus, the base portion 41 and the protruding portion 42 of the plug 40 may be constituted with a flexible material or an elastic material, or for example, only the protruding portion 42 abutting on the inner cavity 11 may be constituted with an elastic material, and the base portion 41 may be constituted with a hard material having no elasticity.

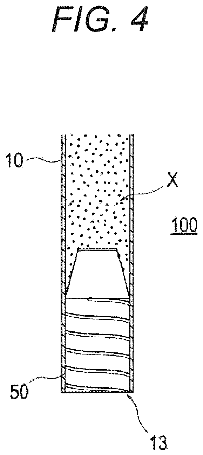

The release portion 50 is disposed on the distal end side of the main body portion 10, and releases the substance to be administered X pushed out by the plug 40 into the living body. The release portion 50 has a plurality of through holes penetrating a plate-like member having a predetermined thickness along an axial direction in order to efficiently administer the substance to be administered X into the living body. The release portion 50 has a plurality of through holes, so that the substance to be administered X can be diffused into the living body without being solidified at one place. Note that a shape and the number of the through holes formed in the release portion 50 can be appropriately determined in consideration of properties of the c X, ease of diffusion of the substance to be administered X at the place where the continuous administration device 100 is indwelled, and the like. In addition, as illustrated in FIG. 4, the release portion 50 may have a shape having one long spiral flow path on the outer peripheral surface of the cylindrical member from the viewpoint of preventing back diffusion. The release portion 50 has the spiral flow path having a length and a flow path width corresponding to a discharge rate of the substance to be administered X, so that it is possible to prevent a biological tissue fluid from being mixed into the substance to be administered X in the second space A2.

The continuous administration device 100 according to the present embodiment has the sliding solid coating layer 60 between the inner cavity 11 of the main body portion 10 and the plug 40.

The sliding solid coating layer 60 reduces initial sliding resistance and normal sliding resistance of the plug 40. The sliding solid coating layer 60 is provided on at least one of the outer peripheral surface of the plug 40 or the inner peripheral surface serving as the region where the plug 40 slides in the inner cavity 11 of the main body portion 10. The sliding solid coating layer 60 may be formed on the outer peripheral surface of the plug 40 at least at a position in contact with the inner peripheral surface of the inner cavity 11 of the main body portion 10. The sliding solid coating layer 60 may be formed at least at a position in contact with the outer peripheral surface of the plug 40 in the region (predetermined region in the second space A2) where the plug 40 slides in the inner cavity 11 of the main body portion 10. In the present embodiment, as illustrated in FIG. 2, the sliding solid coating layer 60 is provided on the entire outer peripheral surface of the plug 40.

In the continuous administration device 100, it is only necessary to shorten at least a period until the drug discharge start time so as to achieve rapid expression of the effect by the administered substance X. Thus, in the continuous administration device 100, in a case where the sliding solid coating layer 60 is formed in the inner cavity 11 of the main body portion 10, the sliding solid coating layer 60 may be formed only in the periphery of a region where the plug 40 stands by upon start of sliding. In this case, a lubricating layer other than the sliding solid coating layer 60 of the present invention such as silicone oil may be provided on an inner cavity forming surface of the inner cavity 11 ahead of the sliding solid coating layer 60. As a result, in the continuous administration device 100, the initial sliding resistance of the plug 40 can be effectively reduced by the sliding solid coating layer 60, and rapid expression of the effect by the administered substance X can be achieved. In addition, in the continuous administration device 100, the initial sliding resistance of the plug 40 can be reduced by the sliding solid coating layer 60 and thus, increase in the internal pressure of the inner cavity 11 until sliding of the plug 40 is started can also be reduced, so that an effect of preventing rapid administration (initial burst) after release of the initial sliding resistance can be expected.

The sliding solid coating layer 60 is constituted with a material capable of obtaining an effect of reducing the initial sliding resistance and the normal sliding resistance of the plug 40. Specifically, the sliding solid coating layer 60 is formed with a composition (hereinafter, simply referred to as a "silicone-based resin composition") containing a silicone-based resin which is formed with a condensate of reactive silicone having a terminal silanol group and has a siloxane bond derived from the silanol group, or a fluorine-based resin film. In a case where the sliding solid coating layer 60 is provided on both the outer peripheral surface of the plug 40 and the inner cavity 11 of the main body portion 10, the sliding solid coating layer 60 may have a configuration in which only one of the silicone-based resin composition or the fluorine-based resin film is employed, or a configuration in which the silicone-based resin composition is provided on one of the outer peripheral surface of the plug 40 or the inner cavity 11 of the main body portion 10 and the fluorine-based resin film is provided on the other.

The silicone-based resin composition includes a composition containing a silicone-based resin including a condensate of reactive silicone having a terminal silanol group and having a siloxane bond derived from the silanol group, and does not contain solid fine particles. As the silicone-based resin composition, those described in WO 2009/084646 A, WO 2012/133264 A, and the like, can be applied as an example.

The composition containing a reactive silicone-based resin is preferably a thermosetting silicone-based resin or a room-temperature curable silicone-based resin, and particularly preferably a thermosetting silicone-based resin from the viewpoint of workability, and the like. The reactive silicone is preferably polydimethylsiloxane having a terminal silanol group. In particular, the reactive silicone preferably has silanol groups at both terminals. In a case where the polysiloxane-based silicone having the terminal silanol group is used as the reactive silicone, the condensate of the reactive silicone has a siloxane bond in the entire main chain.

As the reactive silicone having the terminal silanol group, specifically, a polysiloxane-based silicone having silanol groups at both terminals, such as silanol polydimethylsiloxane at both terminals, silanol polydiphenylsiloxane at both terminals, and silanol diphenylsiloxane-dimethylsiloxane copolymer at both terminals, are suitable. In addition, a form of the reactive silicone is not particularly limited, but it is also possible to use a product obtained by dispersing, emulsifying, and dissolving a polysiloxane composed of the reactive silicone siloxane compound or a condensate thereof as described above in an aqueous medium, a copolymer emulsion obtained by copolymerizing an alkoxysilyl group-containing vinyl monomer with another vinyl monomer as necessary, an emulsion obtained by complexing a polysiloxane with an organic polymer, and the like.

The resin composition that forms the sliding solid coating layer 60 preferably contains a second silicone-based compound different from the reactive silicone-based resin having a silanol group or a siloxane bond. As the second silicone-based compound, alkylalkoxysilane, phenylalkoxysilane, alkylphenoxysilane, aminoalkyl alkoxysilane, glycidoxyalkyl alkoxysilane, or the like, is suitable.

The composition that forms the sliding solid coating layer 60 preferably contains alkyl alkoxysilane or phenyl alkoxysilane as the second silicone-based compound, and further contains aminoalkyl alkoxysilane and/or glycidoxyalkyl alkoxysilane as a third silicone-based compound. More preferably, the resin composition that forms the coating layer contains alkyl alkoxysilane or phenyl alkoxysilane as the second silicone-based compound, further contains aminoalkyl alkoxysilane as the third silicone-based compound, and preferably contains glycidoxyalkyl alkoxysilane as a fourth silicone-based compound.

Further, the second silicone-based compound is preferably alkyl alkoxysilane, alkyl phenoxysilane, phenyl alkoxysilane, or the like. As alkyl alkoxysilane, methyltrimethoxysilane, methyltriethoxysilane, methyltriisobutoxysilane, methyltributoxysilane, methyl sec-trioctyloxysilane, isobutyltrimethoxysilane, cyclohexylmethyldimethoxysilane, diisopropyldimethoxysilane, propyltrimethoxysilane, diisobutyldimethoxysilane, n-octylmethoxysiloxane, ethyltrimethoxysilane, dimethyldimethoxysilane, octyltriethoxysilane, hexyltrimethoxysilane, hexyltriethoxysilane, octamethylcyclotetrasiloxane, methyltri (acryloyloxyethoxy) silane, octyltriethoxysilane, lauryltriethoxysilane, stearyltrimethoxysilane, stearyltriethoxysilane, ethyltriethoxysilane, propyltriethoxysilane, butyltrimethoxysilane, butyltrimethoxysilane, pentyltrimethoxysilane, pentyltrimethoxysilane, heptyltrimethoxysilane, octyltrimethoxysilane, Nonyltriethoxysilane, decyltrimethoxysilane, decyltriethoxysilane, undecyltrimethoxysilane, undecyltriethoxysilane, dodecyltrimethoxysilane, dodecyltriethoxysilane, tridecyltrimethoxysilane, tridecyltriethoxysilane, tetradecyltrimethoxysilane, tetradecyltriethoxysilane, pentadecyltrimethoxysilane, pentadecyltriethoxysilane, hexadecyltrimethoxysilane, hexadecyltriethoxysilane, heptadecyltrimethoxysilane, heptadecyltriethoxysilane, octadecyltrimethoxysilane, nonadecyltriethoxysilane, nonadecyltrimethoxysilane, nonadecyltriethoxysilane, eicosyltrimethoxysilane, eicosyltriethoxysilane, or the like, having at least one alkyl group having 1 to 20 carbon atoms and at least one alkoxy group having 1 to 4 carbon atoms, is suitable.

As alkylphenoxysilane, for example, methyltriphenoxysilane or the like, is suitable. In addition, as phenoxyalkoxysilane, phenyltrimethoxysilane, phenyltriethoxysilane, diphenyldimethoxysilane, diphenyldiethoxysilane, or the like, is suitable.

Furthermore, as the second silicone-based compound, methyltri (glycidyloxy) silane, trimethylchlorosilane, dimethylchlorosilane, methyltrichlorosilane, tetraethoxysilane, heptadecafluorodecyltrimethoxysilane, tridecafluorooctyltrimethoxysilane, tetrapropoxirane, or the like, can also be used.

Furthermore, aminoalkyl alkoxysilane may be used as the second silicone-based compound. As aminoalkyl alkoxysilane, 3-aminopropyltriethoxysilane, 3- (2-aminoethyl) aminopropyltrimethoxysilane, 3- (2-aminoethyl) aminopropylmethyldimethoxysilane, 3-aminopropyltrimethoxysilane, 3-phenylaminopropyltrimethoxysilane, or the like, is suitable.

Furthermore, glycidoxyalkyl alkoxysilane may be used as the second silicone-based compound. As glycidoxyalkyl alkoxysilane, 3-glycidoxypropyltrimethoxysilane, 3-glycidoxypropyltriethoxysilane, 3-glycidoxypropylmethyldiethoxysilane, 3-glycidoxypropylmethyldimethoxysilane, 2- (3,4-epoxycyclohexyl) ethyltrimethoxysilane, or the like, is suitable. Furthermore, as the second silicone compound, a silane-based compound such as 3-ureidopropyltriethoxysilane, diallyldimethylsilane, n-octyldimethylchlorosilane, tetraethoxysilane, or trifluoropropyltrimethoxysilane may be used.

The composition that forms the sliding solid coating layer 60 may contain the second and third silicone-based compounds. The second silicone compound is preferably selected from alkyl alkoxysilanes,
alkylphenoxysilane and phenylalkoxysilane. As the third silicone-based compound, aminoalkyl alkoxysilane or glycidoxyalkyl alkoxysilane is preferably used. Further, the composition that forms the coating layer may contain the second, third and fourth silicone-based compounds. The second silicone compound is preferably selected from alkylalkoxysilane, alkylphenoxysilane, and phenylalkoxysilane. The third silicone-based compound is preferably aminoalkyl alkoxysilane, and the fourth silicone-based compound is preferably glycidoxyalkyl alkoxysilane.

Note that as the reactive silicone-based resin, those described in JP 2007 -244606 A, JP 2009 -532128 A, JP 04144019 B, JP 03430014 B, JP 2002 -505177 A, JP 2006 - 520241 A, JP 04230664 B, US 6663601 A, and the like, can be applied as examples in addition to the above-described materials.

The silicone-based resin composition applicable to the sliding solid coating layer 60 does not contain "solid fine particles". The term "solid fine particles" as used herein refers to particles having a size that affects roughness of the outer surface in a case where the sliding solid coating layer 60 is formed and specifically refers to particles having a particle diameter of more than 10% of the thickness of the sliding solid coating layer 60.

In a case where the above-described silicone-based resin composition is used, the sliding solid coating layer 60 is obtained by dispersing and suspending, in purified water, a material obtained by blending a required amount of the above-described silicone-based resin composition with a required composition on the outer peripheral surface of the plug 40 or the inner peripheral surface of the inner cavity 11 of the main body portion 10 on which the sliding solid coating layer 60 is to be formed, and then applying and curing the coating liquid to the outer surface of the clean plug 40 or the inner peripheral surface of the inner cavity 11. In this event, as a method of applying the coating liquid to the outer surface of the plug 40 or the inner peripheral surface of the inner cavity 11, a method known in related art such as an immersion method or a spraying method can be used. As a curing method, a method of leaving to stand at room temperature may be used, but heat curing is preferable. A thermal curing method is not particularly limited as long as it does not alter or deform a gasket base material, and examples thereof include hot air drying and a drying furnace using infrared rays. Alternatively, a method known in related art such as a method using a vacuum dryer may be used.

The fluorine-based resin film is constituted with a fluorine-based resin that is extremely inert and has chemical resistance, slippage, and adhesion-preventing property.

As the fluorine-based resin film, PTFE (polytetrafluoroethylene), ETFE (ethylenetetrafluoroethylene copolymer), PFE (perfluoroalkoxy alkane), PFA (perfluoroethylene propene copolymer), PVDF (polyvinylidene fluoride), alloys of these with other polymers, or the like, can be applied. In addition, as examples of the fluorine-based resin film, those described in JP 2006-181027 A, JP 2007-54621 A, JP 2008-154644 A, JP 2015-70914 A, JP 2015-150377 A, and the like, can be applied. As a method for coating the plug 40 with the fluorine-based resin film, a known film forming technique can be appropriately adopted.

Further, as the sliding solid coating layer 60, in addition to the silicone-based resin composition and the fluorine-based resin film described above, a parylene coat (one example is disclosed in JP 2002-177364 A), a diamondlike carbon (DLC) coat (one example is disclosed in JP 2001-190665 A), a coating material (as one example, US 10537273 B) produced from an organosilicon precursor, or the like, can be appropriately selected.

As described above, the continuous administration device 100 according to the present embodiment has the sliding solid coating layer 60 produced by appropriately using the above-described material on at least one of the outer peripheral surface of the plug 40 or the inner peripheral surface serving as the region where the plug 40 slides in the inner cavity 11 of the main body portion 10. When sliding at a low speed (1 mm/min or less), an extremely low speed (0.01 mm/min or less), or a super extremely low speed (0.0015 mm/min) or lower, the sliding solid coating layer 60 has an effect of significantly reducing initial sliding resistance and normal sliding resistance as compared with silicone oil. A sustained release rate of the continuous administration device 100 is a rate for releasing the substance to be administered X (drug) filled in the main body portion 10 by a certain amount for a predetermined period, and can be a value obtained by dividing an entire length of a region (corresponding to the region where the plug 40 slides) filled with the substance to be administered X by an administration period (minute). For example, in a case where a total length of the region filled with the substance to be administered X is 30 mm, it is "0.0015 mm/min" in the case of sustained release for two weeks (elapsed minutes: 20160 minutes), "0.0001 mm/min" in the case of sustained release for 180 days (elapsed minutes: 259200 minutes), and "0.00001 mm/min" in the case of sustained release for five years (elapsed minutes: 2628000 minutes). As described above, in the continuous administration device 100, in order to sustainably release the substance to be administered X over a long period of time, it is necessary to slide the plug 40 at a sliding speed of a super extremely low speed or lower.

Silicone oil generally used as a lubricant can significantly reduce the normal sliding resistance of the plug 40 as compared with the above-described various materials applicable to the sliding solid coating layer 60 according to the present embodiment at an administration rate in a relatively short period (about several seconds to several tens of minutes) such as bolus administration. On the other hand, as in the continuous administration device 100 according to the present embodiment, various materials applicable to the sliding solid coating layer 60 can significantly reduce the initial sliding resistance and the normal sliding resistance of the plug 40 as compared with silicone oil, at a low speed, an extremely low speed, or a super extremely low speed for continuous administration for a long period of time such as several weeks to several months or several years, as described in Examples described later. Such a reverse phenomenon of the sliding resistance reduction effect depending on the administration rate (sliding speed of the plug 40) is a phenomenon that the present inventors have found from examination results of Examples described in detail later through search for a material that can significantly reduce the initial sliding resistance and the normal sliding resistance of the plug 40.

As a result of the continuous administration device 100 including the sliding solid coating layer 60, the initial sliding resistance and the normal sliding resistance of the plug 40 are reduced and a period until the drug discharge start time is shortened in a case where the substance to be administered X is continuously administered at an administration rate of a low speed to an extremely low speed and a super extremely low speed for a long period of time. Thus, the continuous administration device 100 can accelerate time until the effect by the administration of the administered substance X is expressed.

### <Operation>

Next, an operation example of the continuous administration device 100 according to the present embodiment will be described with reference to FIGS. 3A to 3D.

The continuous administration device 100 is indwelled in the living body after being filled with the substance to be administered X to the administration target. In the continuous administration device 100, as illustrated in FIG. 3A, a state of the continuous administration device 100 is a state before the liquid component of the body fluid permeates the liquid permeation portion 20, and thus, the pressing portion 30 is not expanded, and sliding of the plug 40 is not started.

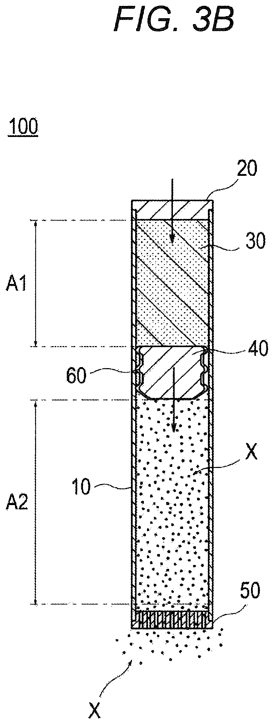

As illustrated in FIG. 3B, if a predetermined period elapses after the continuous administration device 100 is indwelled in the living body, the liquid component in the body fluid permeates into the liquid permeation portion 20. The pressing portion 30 starts to expand by the liquid component that has permeated from the liquid permeation portion 20. The plug 40 starts sliding toward the downstream side of the second space A2 as a result of the internal pressure of the inner cavity 11 increasing as the pressing portion 30 expands. In the continuous administration device 100, the sliding solid coating layer 60 is provided on the outer peripheral surface of the plug 40, and thus, the initial sliding resistance is significantly reduced, and the plug 40 starts to slide in a short period of time.

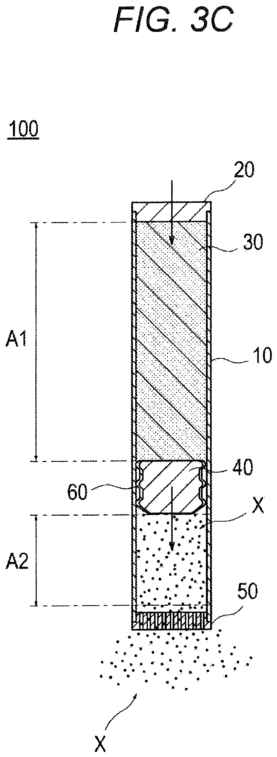

As illustrated in FIG. 3C, in the continuous administration device 100, if sliding shifts to normal sliding after initial sliding,
the plug 40 gradually slides to the downstream side. In this event, the plug 40 slides to the downstream side at a substantially constant sliding speed so that a prescribed amount of the substance to be administered X is continuously administered for a long period of time.

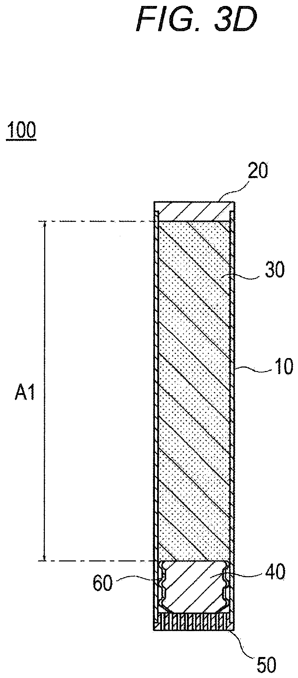

Then, as illustrated in FIG. 3D, in the continuous administration device 100, sliding of the plug 40 is stopped in accordance with end time of the administration period of the substance to be administered X. As illustrated in FIG. 3D, a distal end of the plug 40 is in contact with a surface on the proximal end side of the release portion 50. If the administration period of the substance to be administered X ends, the continuous administration device 100 is extracted from the living body.

### [Operational Effects]

As described above, the continuous administration device 100 according to the present embodiment is a device that includes the cylindrical main body portion 10 having the inner cavity 11, the plug 40 slidable in the inner cavity 11 in a liquid-tight manner, the pressing portion 30 disposed on the upstream side with respect to the plug 40 in the inner cavity 11 and pressing the plug 40 toward the downstream side, and the substance to be administered X stored on the downstream side with respect to the plug 40 in the inner cavity 11 and released into the living body by the plug 40 sliding toward the downstream side, and is indwelled in the living body for sustained release of the substance to be administered X. The continuous administration device 100 includes the sliding solid coating layer 60 formed with a silicone-based resin composition or a fluorine-based resin film for reducing initial sliding resistance and normal sliding resistance of the plug 40, on at least one of the outer surface of the plug 40 or the region where the plug 40 slides in the inner cavity 11.

The sliding solid coating layer 60 has an effect of significantly reducing initial sliding resistance and normal sliding resistance as compared with silicone oil when sliding is performed at a low speed, an extremely low speed, or a super extremely low speed. Thus, in a case where the continuous administration device 100 continuously administers the substance to be administered X at a low speed, an extremely low speed, or a super extremely low speed for a long period of time, the initial sliding resistance and the normal sliding resistance of the plug 40 are reduced, and a period until the drug discharge start time is shortened. Thus, the continuous administration device 100 can accelerate time until the effect by the administration of the administered substance X is expressed.

Note that the continuous administration device 100 according to the present embodiment described above has been described with a configuration in which the osmotic engine that expands by the liquid component of the body fluid in the living body is used as the configuration of the pressing portion 30 that presses the plug 40. However, the configuration of the pressing portion 30 is not limited to the osmotic engine, and for example, a mechanical configuration using a motor as a drive source can also be adopted. Even in the continuous administration device 100 equipped with such a mechanical pressing portion 30, the sliding solid coating layer 60 is provided on one of the outer surface of the plug 40 or the region where the plug 40 slides in the inner cavity 11, so that the effect of significantly reducing the initial sliding resistance and the normal sliding resistance of the plug 40 can be obtained.

### [Examples]

Hereinafter, the present invention will be specifically described with reference to Examples, but the scope of the present invention is not limited to the following Examples.

### [Examination 1]

In Evaluation Examination 1, initial sliding resistance and normal sliding resistance were compared between silicone oil that can be generally applied as a lubricant and a silicone-based resin composition and a fluorine-based resin film that can be applied to the sliding solid coating layer according to the present embodiment described above.

### <Examination Conditions>

As an examination instrument, a tensile/compression tester "EZ-test (manufactured by Shimadzu Corporation)" was used. As the main body portion, a 1 ml syringe "PLAJEX Luer lock type (manufactured by TERUMO CORPORATION)" equipped with a gasket (corresponding to the plug) having a peak (corresponding to the protruding portion) was used in a state where a needle was not attached.

As a lubricant coated on the outer surface of the gasket of the syringe, in Example 1,
a silicone-based resin composition applicable to the sliding solid coating layer according to the present embodiment was used, and in Example 2, a fluorine-based resin film applicable to the sliding solid coating layer according to the present embodiment was used. In Comparative Example 1, silicone oil "360 Medical Fluid 12500 (manufactured by Dow Corning Toray Co., Ltd.)" was used. Each sample corresponding to Example 1, Example 2, and Comparative Example 1 was prepared by coating the gasket with each of these lubricants and then capping the gasket. Note that in Comparative Example 1, storage was performed under severe conditions (60 °C for one week) in order to reproduce a sticking state which is a problem of the silicone oil.

An examination speed (pressing speed) is a speed at which a pusher of the syringe is pressed. The examination speed was set to three speeds of an extremely low administration rate "speed 1: 0.01 mm/min" for the purpose of long-term sustained release, a normal administration rate "speed 3: 200 mm/min" corresponding to bolus administration, and a low administration rate "speed 2: 1 mm/min" which is intermediate between speed 1 and speed 3.

Table 1 indicates results of initial sliding resistance value (N) and normal sliding resistance value (N) at each examination speed in Example 1, Example 2, and Comparative Example 1. The "initial sliding resistance" in the table is a maximum value (upper yield point) when the plug yields while the plug starts sliding and shifts to normal sliding. In addition, the "normal sliding resistance" in the table is a maximum value when sliding of the plug shifts to the normal sliding after the initial sliding.

**[Table 1]**

| | Speed 1 (0.01 mm/min) | | Speed 2 (1 mm/min) | | Speed 3 (200 mm/min) | |
|---|---|---|---|---|---|---|
| | Initial sliding (N) | Normal sliding (N) | Initial sliding (N) | Normal sliding (N) | Initial sliding (N) | Normal sliding (N) |
| Example 1 | 1.5 | 1.4 | 3.5 | 3.8 | 5.1 | 11.4 |
| Example 2 | 2..7 | 8.1 | 3.4 | 10.2 | 8.0 | 18.8 |
| Comparative Example 1 | 8.3 | 13.3 | 14.8 | 10.6 | 35.8 | 2.0 |

### <Results>

As indicated in Table 1, in a case where sliding was performed at speed 1 in Example 1, Example 2, and Comparative Example 1,

The initial sliding resistance of Example 1 and Example 2 was smaller than the initial sliding resistance of Comparative Example 1, and the normal sliding resistance was also smaller than that of Comparative Example 1. In a case where sliding was performed at speed 2 in Example 1, Example 2, and Comparative Example 1, the initial sliding resistance of Example 1 and Example 2 was smaller than the initial sliding resistance of Comparative Example 1, and the normal sliding resistance was also smaller than that of Comparative Example 1. On the other hand, in a case where sliding was performed at speed 3 in Example 1, Example 2, and Comparative Example 1, the initial sliding resistance of Example 1 and Example 2 was smaller than the initial sliding resistance of Comparative Example 1, and the normal sliding resistance was higher than that of Comparative Example 1.

From the above, it was confirmed that in the case of an administration rate for performing sustained release over a relatively long period of time, such as speed 1 or speed 2, the initial sliding resistance and the normal sliding resistance can be significantly reduced in Example 1 and Example 2 as compared with Comparative Example 1. In addition, it was confirmed that in the case of a normal administration rate such as bolus administration at speed 3, the normal sliding resistance can be significantly reduced in Comparative Example 1 as compared with Examples 1 and 2. As indicated in Table 1, the sliding solid coating layer exhibits an effect of reducing initial sliding resistance by about 70% as compared with silicone oil. It has been therefore proved that both the silicone-based resin composition and the fluorine-based resin film, which are materials applicable to the sliding solid coating layer according to the present embodiment, significantly reduce the initial sliding resistance and the normal sliding resistance of the plug at a low administration rate of 1 mm/min or less and an extremely low administration rate.

### [Examination 2]

In Evaluation Examination 2, a period until the sliding start time of the plug was compared when the silicone oil that can be generally applied as a lubricant, the silicone-based resin composition and the fluorine-based resin film applicable to the sliding solid coating layer according to the present embodiment described above, were applied to the plug (gasket).

### <Examination Conditions>

As a container sample serving as the main body portion, a container sample having the same specification as the syringe used in Examination 1 was used. As a lubricant for coating the outer surface of the gasket of the syringe, a silicone-based resin composition "i-coating (manufactured by Terumo Corporation)" applicable to the sliding solid coating layer according to the present embodiment was used as Example 1, and a fluorine-based resin film applicable to the sliding solid coating layer according to the present embodiment was used as Example 2. Further, as Comparative Example 1, silicone oil "360 Medical Fluid 12500 (manufactured by Dow Corning Toray Co., Ltd.)" was used. Note that in Comparative Example 1, storage was performed under severe conditions (60 °C for one week) in order to reproduce a sticking state which is a problem of the silicone oil.

After Example 1, Example 2, and Comparative Example 1 were applied as lubricants to the outer surface of the gasket, the gasket was capped with the syringe. Sodium chloride was filled at a terminal of the syringe as the pressing portion (osmotic engine), and then the syringe was filled with PEG 400 (polyethylene glycol 400) to eliminate air in the syringe. Each sample was obtained by plugging the terminal of the syringe with a semipermeable membrane "Winding CELLOFAN (manufactured by HEICO PACK CO., LTD.)" as the liquid permeation portion. These samples were immersed in water at normal temperature, and a period until the sliding start time of the gasket was measured.

Table 2 indicates results of periods until the sliding start time (min) in Example 1, Example 2, and Comparative Example 1.

**[Table 2]**

| | Period until sliding start time (min) |
|---|---|
| Example 1 | 44.5 |
| Example 2 | 54.8 |
| Comparative Example 1 | 140.9 |

### <Results>

As indicated in Table 2, the period until the sliding start time (Example 1: 44.5 min, Example 2: 54.8 min) of the gasket in each of Example 1 and Example 2 was shortened to about 1/3 as compared with the period until the sliding start time (140.9 minutes) of Comparative Example 1. From the above, it was confirmed that Example 1 and Example 2 significantly reduced the initial sliding resistance as compared with Comparative Example 1. Thus, it has been proved that the silicone-based resin composition and the fluorine-based resin film, which are materials applicable to the sliding solid coating layer according to the present embodiment, significantly reduce the initial sliding resistance of the plug.

### [Examination 3]

In Evaluation Examination 3, the silicone oil that can be generally applied as a lubricant and the silicone-based resin composition applicable to the sliding solid coating layer according to the present embodiment described above were compared using a main body portion and a piston (plug) of a size to be used in an actual administration device.

### <Examination Conditions>

As an examination instrument, a tensile/compression tester "EZ-test (manufactured by Shimadzu Corporation)" was used. As the main body portion, a metal pipe including a release portion at a distal end and a gasket (corresponding to the plug) having a peak (corresponding to the protruding portion) on the outer peripheral surface was used. Materials and dimensions of each component are indicated below.
- Pipe (material: 64 titanium alloy, total length: 45 mm, outer diameter: 4 mm, inner diameter: 3 mm)
- Gasket (material: chlorinated butyl rubber (CIIR))
- Release portion (material: high density polyethylene (HDPE), flow path inner diameter: 0.15 mm, spiral flow path length: 30 mm)

As the lubricant with which the outer surface of the gasket of the pipe was coated, the silicone-based resin composition applicable to the sliding solid coating layer according to the present embodiment was used in Example 3, and silicone oil "360 Medical Fluid 12500 (manufactured by Dow Corning Toray Co., Ltd.)" was used in Comparative Example 2. Each of samples corresponding to Example 3 and Comparative Example 2 was prepared by coating the gasket with each of these lubricants and then capping the gasket. In Comparative Example 2, storage was performed under severe conditions (60 °C for one week) in order to reproduce a sticking state which is a problem of the silicone oil.

An examination speed (pressing speed) was set to "speed 4: 0.001 mm/min", which is the lowest speed that can be implemented by the examination equipment.

Table 3 indicates results of an initial sliding resistance value (N) and a normal sliding resistance value (N) at each examination speed in Example 3 and Comparative Example 2. The "initial sliding resistance" and the "normal sliding resistance" in the tables are synonymous with the respective terms indicated in Table 1.

**[Table 3]**

| | Speed 4 (0.001 mm/min) | |
|---|---|---|
| | Initial sliding (N) | Normal sliding (N) |
| Example 3 | 2.3 | 0.6 |
| Comparative Example 2 | 3.6 | 4.5 |

### <Results>

As indicated in Table 3, in a case where sliding was performed at speed 4 with Example 3 and Comparative Example 2, the initial sliding resistance of Example 3 was smaller than the initial sliding resistance of Comparative Example 2, and the normal sliding resistance was also smaller than that of Comparative Example 2.

From the above, it was confirmed that even in a case where speed 4 slower than speed 1 in Examination 1 was used as the administration rate, Example 3 significantly reduced the initial sliding resistance and the normal sliding resistance as compared with Comparative Example 2. As indicated in Table 3, the sliding solid coating layer exhibits an effect of reducing initial sliding resistance by about 60% as compared with silicone oil. Thus, it has been proved that the silicone-based resin composition which is a material applicable to the sliding solid coating layer according to the present embodiment significantly reduces the initial sliding resistance and the normal sliding resistance of the plug at an extremely low administration rate of 0.001 mm/min or less.

The present application is based on Japanese Patent Application No. 2021-051366 filed on March 25, 2021, the disclosure content of which is incorporated herein by reference in its entirety.

### Reference Signs List

- 10: Main body portion
- 11: Inner cavity
- 20: Liquid permeation portion
- 30: Pressing portion
- 40: Plug
- 50: Release portion
- 60: Sliding solid coating layer
- 100: Continuous administration device
- A1: First space
- A2: Second space
- X: Substance to be administered

## Claims

1. A continuous administration device comprising:
a cylindrical main body portion having an inner cavity;
a plug slidable in the inner cavity in a liquid-tight manner;
a pressing portion disposed on an upstream side with respect to the plug in the inner cavity and pressing the plug toward a downstream side; and
a substance to be administered stored on the downstream side with respect to the plug in the inner cavity and released into a living body by the plug sliding toward the downstream side, and
the continuous administration device being indwelled in the living body for sustained release of the substance to be administered,
wherein the continuous administration device comprises a sliding solid coating layer for reducing initial sliding resistance and normal sliding resistance of the plug, on at least one of an outer surface of the plug or a region where the plug slides in the inner cavity.

2. The continuous administration device according to claim 1, wherein the sliding solid coating layer includes a silicone-based resin composition or a fluorine-based resin film.

3. The continuous administration device according to claim 2, wherein the silicone-based resin composition is formed with a composition containing a silicone-based resin formed with a condensate of reactive silicone having a terminal silanol group and having a siloxane bond derived from the silanol group, and does not contain solid fine particles.

4. The continuous administration device according to claim 3, wherein the silicone-based resin composition further contains alkylalkoxysilane as a second silicone-based compound different from the silicone-based resin having the siloxane bond, aminoalkyl alkoxysilane as a third silicone-based compound, and further glycidoxyalkyl alkoxysilane as a fourth silicone-based compound.

5. The continuous administration device according to claim 1, wherein the pressing portion presses the plug so as to slide at 1 mm/min or less.

6. The continuous administration device according to claim 1, wherein the pressing portion presses the plug so as to slide at 0.01 mm/min or less.

7. The continuous administration device according to claim 1, wherein the pressing portion presses the plug so as to slide at 0.0015 mm/min or less.
